# EUROPEAN PATENT APPLICATION

(11) **EP 2 149 567 A1**
(43) Date of publication of application: **03.02.2010**
(21) Application number: 08160684.0
(22) Date of filing: 18.07.2008
(51) Int. Cl.: C07D 257/02, C07D 405/12, C07K 5/02, A61K 51/04, A61K 51/08, A61P 35/00

(54) **Cyclic polyamines for binding phosphatidylserine**

(71) Applicant: Bayer Schering Pharma Aktiengesellschaft, 13353 Berlin (DE)
(72) Inventor: Zitzmann-Kolbe, Sabine, 10551, Berlin (DE); Oltmanns, Dörte, 69469 Weinheim (DE); Eisenhut, Michael, 69118 Heidelberg (DE); Bauder-Wüst, Ulrike, 69198 Schreisheim (DE)

(57) **Abstract**

The invention pertains to a compound for binding and detecting phosphatidylserine. According to the invention, the compound comprises a cyclic polyamine unit for the complexation of a bi- or trivalent metal ion. The compound of the invention may contain a reporting tag like fluorescein or a radioisotope including substituent.

## Description

The invention pertains to a compound for binding phosphatidylserine (PS), and its use for binding phosphatidylserine in vitro and in vivo, as well as to a method for detecting the progression or regression of a tumor in a patient. Furthermore, the invention relates to a pharmaceutical composition, comprising such a compound, and to a kit for binding phosphatidylserine.

Throughout this application, various publications are cited. The disclosure of these publications is hereby incorporated by reference in its entirety into this application to describe more fully the state of the art to which this invention pertains.

### Introduction

Therapy control of tumors is one of the main areas of interest during the follow up of cancer patients. This information is currently acquired by a series of conventional investigations including morphological changes, symptomatic responses and clinical chemistry. Radiological techniques like X-ray, computed tomography (CT), ultrasonography (US) and magnetic resonance (MR) are imaging techniques which cover the size and morphology of tumor masses and to some degree alterations of tumor perfusion.

At the same time, nuclear medicine has a series of radiopharmaceuticals at its disposal which are successfully used for the assessment of therapy response. Among those agents, [¹⁸F]FDG represents the main protagonist which is acting as a surrogate marker used to show the influence of e.g. cytostatic drugs on the glucose transport and hexokinase activity in tumors. A decrease in tracer accumulation is expected to indicate treatment response and a better survival. However, interpretation of the signal might be complicated by inflammatory reactions, for example early after radiation therapy. Furthermore, tumor response is shown via a negative signal, i.e. a decrease in tracer uptake. This may be problematic in cases of a moderate accumulation of the tracer prior to therapy. In this respect, tracers providing a positive signal are more desirable.

A few years ago, ^{99m}Tc labeled annexin V was presented as a new agent capable to image cell death in vivo. Apoptosis was visualized in several model systems including myocardial infarction, Fas antibody treated liver and tumors after cytostatic drug application (1, 2). The use of ^{99m}Tc labeled annexin V as an in vivo imaging indicator for apoptosis based on results obtained with the annexin V - fluorescent dye conjugate was used to show apoptotic cells under the microscope (3).

Annexin V has first been isolated from human placenta (4) demonstrating an anticoagulant effect by inhibiting among other factors prothrombinase activity. This effect was attributed to the displacement of coagulation factors from the phospholipid membrane (5).

Annexins are ubiquitous homologous proteins that bind to phospholipids in the presence of calcium. Annexin V, formerly also named as placental anticoagulant protein (PAP) or vascular anticoagulant protein (VAC-α) (5), binds together with Ca²⁺ on externalized phosphatidylserine (PS) present on the cell membranes early after the onset of apoptosis. Under normal conditions, phosphatidylserine resides to about 100 % on the interior leaflet of the bi-layered cell membrane where it functions as an anchor for e.g. annexin I, which is involved in transmembrane signaling and internalization processes (6). Due to the activation of effector caspases 3, 6 and 7, which are induced either after intrinsic or extrinsic stimuli, proteolytic inactivation of proteins occurs which maintain under normal conditions the asymmetric distribution of lipids within the cell membrane. Several types of translocase enzymes belong to these proteins, including an ubiquitous Mg²⁺-ATP dependent aminophospholipid flippase that selectively catalyzes the inward transport of phosphatidylserine and phosphatidylethanolamine, an ATP dependent floppase that moves phospholipids outward with little headgroup specificity, and a Ca²⁺ dependent scramblase that destroys the bilayer asymmetry (7). ATP depletion due to the energy demanding apoptotic process may also hamper the maintenance of the asymmetric lipid distribution.

The high affinity of annexin V for cells which expose phosphatidylserine, exhibiting a K_{d} of <10⁻¹⁰ mol/l (8) is the basis of detecting apoptosis in vivo. The structural background for this strong annexin V - phosphatidylserine interaction is slowly elucidated.

It is known from X-ray structure analyses that this kind of binding functions through the co-complexation of Ca²⁺ with the carboxyl-phospholipid headgroup in collaboration with so called AB, AB' and DE Ca²⁺-binding sites. These binding sites are located in each of the four tandemly repeated segments of annexin V.

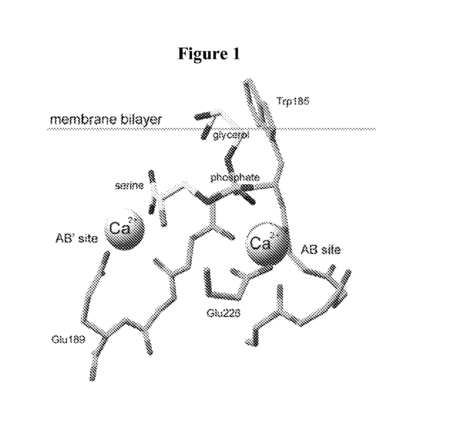

Figure 1 illustrates the interaction in one of these segments (domain III) with glycerophosphoserine (truncated phosphatidylserine) on Ca²⁺-annexin V binding sites (9). Primary AB and secondary AB' Ca²⁺ sites are located within the loop between the A and B helices of each domain. They are also called type II and type III Ca²⁺ binding sites, respectively.

It should be noted that the three complexation sites AB, AB' and DE imply differently coordinated calcium cations with different stabilities, namely AB > AB' > DE. The second complex entity phosphatidylserine coordinates Ca²⁺ in the AB' site with the carboxyl function of serine and with the negatively charged phosphate oxygen in the AB site. In addition, the α-amino group of serine interacts in this domain with Thr 187.

The interaction of annexin V with phosphatidylserine through jointly used Ca²⁺ is, however, not alone responsible for the strong binding affinity. Multivalent binding must be taken into account, since Ca²⁺ is forming peptide complexes with a stability constant of log K₁ in the range of only 4-5. In combination with phosphatidylserine, this value may increase to some degree because of the bidenticity of phosphatidylserine for Ca²⁺. However, in order to meet the above mentioned small dissociation constant, two or more domains must be involved in the annexin V / Ca²⁺ / phosphatidylserine headgroup interactions. These considerations assume that Ca²⁺ is present in a sufficient concentration. The Ca²⁺ dependency was validated by titration in presence of annexin V binding at low membrane occupancy showing strong cooperativity of this interaction with respect to Ca²⁺ (10). Up to 12 Ca²⁺ ions can theoretically be complexed by three binding sites (AB, AB' and DE) in each of the domains. As many as 10 Ca²⁺ ions have been located in rat annexin V crystal structures all binding at the membrane-facing surface of the protein (9).

Besides the annexin V/phosphatidylserine system, other approaches have been tested for the imaging of apoptosis. Alternative targets in the apoptotic pathway potentially useful for the in vivo detection of apoptosis are activated caspases (11-13). The two systems tested in this context are based on the binding of irreversible inhibitors or substrates to activated executioner caspases. The hitherto obtained results have been summarized recently (14, 15).

As already mentioned before, annexin V is typically labeled with a fluorescent dye for *in vitro* assays (3). Although it is utilized extensively in molecular biology, the labeled protein is expensive and moderately unstable. Thus, it is not convenient for high throughput screening assays in drug discovery.

Additionally, approximately 2.5 mmol/l of extracellular Ca²⁺ is needed for complete binding. This can lead to false positive results because most animal cells have a Ca²⁺ dependent scramblase that can move phosphatidylserine to the cell surface (16). Furthermore, complete annexin binding requires incubation times of up to one hour, which is problematic for kinetic assays (17, 18).

In short, dye labeled annexin V is a useful apoptosis sensor, but it has a number of limitations and there is a need for replacement reagents that are cheap, robust, low-molecular weight, rapidly-binding, membrane-impermeable, and Ca²⁺-independent. Accordingly, the problem underlying the present invention was to provide a molecule that would show improved characteristics as a phosphatidylserine-binding agent and, in particular, to provide a molecule that would show improved characteristics as an apoptosis sensor over annexin V.

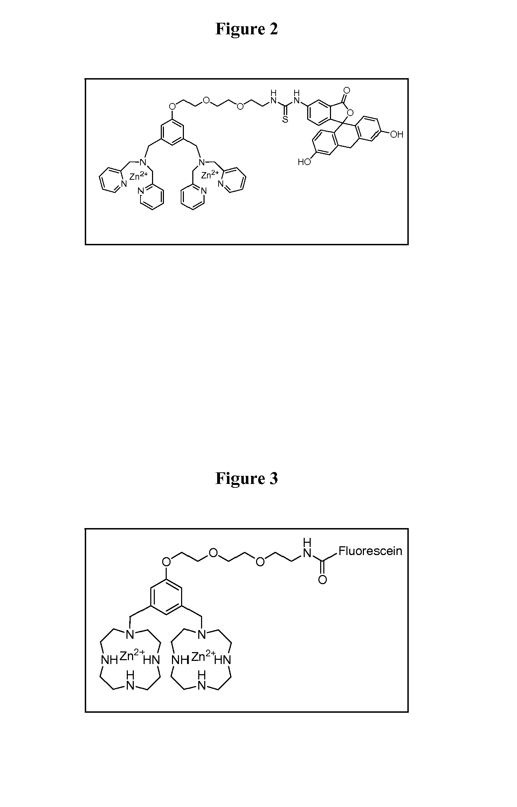

The recently developed zinc bis-(2,2-dipicolylamine) (Zn²⁺ bis-DPA) coordination complex shown in Figure 2 has been designed to mimic the apoptosis sensing function of annexin V (19, 20). According to Figure 1, one phosphatidylserine head group is coordinated to two bridging Ca²⁺ ions that are in turn coordinated to one of the four canonical binding sites at the protein surface (9).

### Description of the invention

Contrary to the expectation that one of the Zn-coordinating heteroatoms has to be water in order to provide a replacable binding site for phosphatidylserine, the inventors used a compound with at least one cyclic polyamine unit for the complexation of a bi- or trivalent metal ion, in particular cyclen (12[ane]N₄) or cyclam (14[ane]N₄) instead of DPA. Cyclen is a cyclic ethylene bridged tetramine, and cyclam is a mixed ethylene-propylene bridged tetramine, both of which have four nitrogen atoms for Zn²⁺ complexation. The stability constant for this class of compounds is about 10¹⁵ (mol/l)⁻¹ (for cyclam), as compared to 10⁵ (mol/l)⁻¹ for DPA.

Accordingly, in one aspect of the invention, the underlying problem is solved by a compound or complex for binding and detecting phosphatidylserine, that comprises or contains at least one cyclic polyamine unit. This cyclic polyamine unit allows for the binding or complexation of a multivalent, in particular of a bi- and/or a trivalent metal ion as a central ion of the complex.

Excluded from the scope of the invention, in one aspect of the invention, is AMD3100 (1,1'-[1,4-Phenylenebis(methylen)]bis [1,4,8,11-tetraazacyclotetradecan]-octohydrobromid-dihydrate) as such and its derivatives (i.e. substituted AMD3100), which is a compound with cyclic polyamine units. AMD 3100 was designed to treat HIV infections by inhibiting the viral entry through the CD4 / CXCR4 receptor assembly. The use of AMD3100 for binding phosphatidylserine and for apoptosis imaging, however, lies within the scope of the invention.

When the multivalent metal ion is a bivalent metal ion, this bivalent metal ion is chosen from the group consisting of Zn²⁺, Ca²⁺, C²⁺, Be²⁺, Mg²⁺, Sr²⁺ Ba²⁺, Cd²⁺, Cr²⁺ Mn²⁺, Fe²⁺, Co²⁺, Ni²⁺, Pb²⁺, and Hg²⁺, as well as the corresponding radiometals of all of the aforementioned bivalent metal ions.

When the multivalent metal ion is a trivalent metal ion, this trivalent metal ion is chosen from the group consisting of Al³⁺ Ga³⁺, In³⁺, Cr³⁺, Mn³⁺, Fe³⁺, Co³⁺, Ni³⁺, as well as the corresponding radiometals of all of the aforementioned trivalent metal ions.

In embodiments in which the compound of the invention comprises more than one cyclic polyamine unit, different metal ions can be complexed by the compound.

In order to facilitate easy detection of the compound, e.g. after administration to a patient or when used in an in vitro detection method for detecting phosphatidylserine, the compound further comprises at least one a detectable tag (such as a label or signalling group) for detecting the compound, e.g. in the patient. This detectable tag is preferably covalently bound to the compound.

Such a tag can be chosen from the group consisting of fluorescent dyes, enzymes (in particular for the use in an ELISA assay), proteins, and radioactive labels.

In particular, the fluorescent dye can be, for example, a fluorescein, a rhodamine, a BODIPY fluorophore (4,4-difluoro-4-bora-3a,4a-diaza-s-indacene, Molecular Probes), an Alexa Fluor dye (Molecular Probes), Cy-3, Cy-5, and derivatives thereof.

The protein can be, for example, a peroxidase, luziferase, biotin, avidin, an antibody and/or a part of an antibody.

The radioactive label can be chosen from the group consisting of ^{99m}Tc, ¹⁸F, ¹¹C ¹²³I, ¹²⁴I, ¹³¹I, ⁶⁴Cu²⁺, ⁶⁸Ga³⁺, ¹¹¹In³⁺, ¹⁴C, ³H, ³²P, and ³³P. This radioactive label can be the complexed metal ion as described above and/or can be a group or atom that is covalently bound to the compound.

In particular, for positron emission tomography (PET), ¹⁸F, ⁶⁸Ga, ⁶⁴Cu or ¹²⁴I are preferred as positron emitting radioisotopes. For single-photon emission computed tomography (SPECT), ¹²³I_{,} ¹¹¹In_{,} and ^{99m}Tc are preferred.

Besides detectable tags, the compound can also be covalently or non-covalently bound to therapeutic compounds, such as drugs, like antiapoptotic substances which can be administered to a patient, for example after a stroke.

In a preferred embodiment of the invention, the at least one cyclic polyamine unit of the compound is cyclen (an ethylene bridged tetramine) or cyclam (a mixed ethylene-propylene bridged tetramine). Preferably, the invention compound comprises one to four cyclic polyamine units. More preferably, the invention compound comprises one to two cyclic polyamine units. The structure of cyclen is shown as formula Ia, and the structure of cyclam is shown as formula Ib.

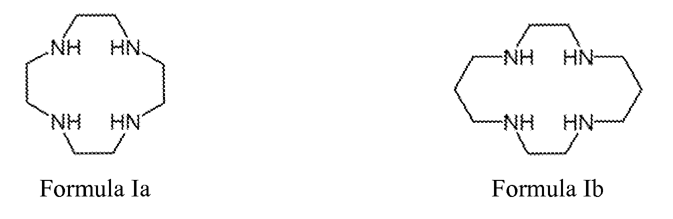

As described above, a multivalent (in particular a bi- or trivalent) metal ion can be complexed by a cyclen or a cyclam ring through the ring's N atoms.

In a preferred embodiment, the compound contains or comprises at least two cyclic polyamine units that are connected via a bridging unit. This bridging unit connects the at least two cyclic polyamine units with each other. As a bridging unit, several structures are possible. Below, preferred bridging units are shown:

In one preferred embodiment of the compound of the invention, the bridging unit can be a unit according to formula II. 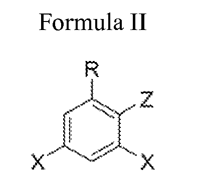 with
X = (CH₂)ₙ, (CH₂CH₂O)ₙ, CH₂OPhCH₂, (CH₂CH₂CH₂O)ₙ, (CONHPhCH₂), (CONH(CH₂CH₂O)₂CH₂CH₂), or CO,
wherein Ph = phenyl and n = 1, 2, or 3; and
wherein X is covalently bound to a cyclic polyamine unit, such as cyclen or cyclam (in particular to one of its N atoms); and
Z = a halogen (such as F, Cl, Br, or I), or a radiohalogen (such as ¹⁸F, ¹²³I), or H;
R = a detectable tag as described above.

Preferably, the detectable tag R is or comprises a fluorescent dye, a radioisotope, an enzyme, a protein, or a labeled substituent. More preferably, R is a labeled substituent R₂, which optionally is linked to the phenyl group via a spacer R₁. The spacer R₁ (liked to labeled substituent R₂) is preferably of one of the following formulas 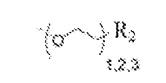 or

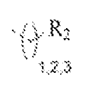

The labeled substituent R₂ is preferably of one of the following formulas 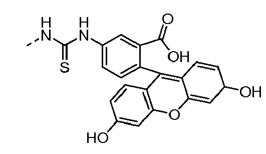 (FITC-tag) or 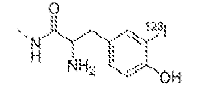 or 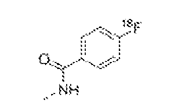 or

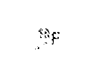

Preferably, if Z is a radiohalogen, such as ¹⁸F or ¹²³I, then R is O-alkyl or OH in a compound of the invention with a bridging unit of formula II. Furthermore, it is preferred that both X in formula II are identical, although they could also differ from each other.

In another preferred embodiment, the bridging unit can be a unit according to formula III or formula IIIa, which is a substituted benzene-based scaffold. 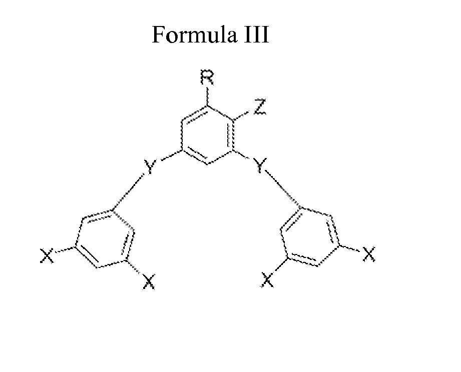 with X = (CH₂)ₙ, (CH₂CH₂O)ₙ, CH₂OPhCH₂, (CH₂CH₂CH₂O)ₙ, (CONHPhCH₂), (CONH(CH₂CH₂O)₂CH₂CH₂), or CO,
wherein Ph = phenyl and n = 1, 2, or 3, and
wherein X is covalently bound to a cyclic polyamine unit, such as cyclen or cyclam; and
Y = (CH₂)ₙ (with n=1, 2, or 3), (CH₂CH₂O)ₘ (with m=0, 1, 2, or 3), O(CH₂)ₚO (with p=1, 2, 3, or 4), or O (independently of each other), and
Z = a halogen (such as F, Cl, Br, or I), or a radiohalogen (such as ¹⁸F, ¹²³I), or H; and
R = a detectable tag as described above, in particular a fluorescent dye, a radioisotope, or a labeled substituent, e.g. a labeled substituent R₂, which optionally is linked to the phenyl group via a spacer R₁. The spacer R₁ is preferably of one of the following formulas (liked to labeled substituent R₂) 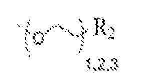 or

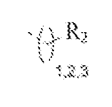

The labeled substituent R₂ is preferably of one of the following formulas 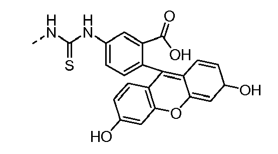 or 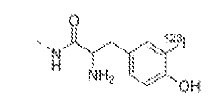 or 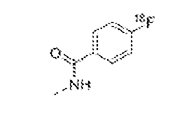 or

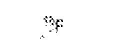

In one embodiment, in formula III, X can be bound to the phenyl group via a group Y as defined above, instead of being covalently bound to the phenyl group directly. Examples for this embodiment can be found in table 1 below.

In formula III, if Z is a radiohalogen, such as ¹⁸F, ¹²³I, then R is preferably O-alkyl, O-aryl, or OH.

In another preferred embodiment, the bridging unit can be a unit according to formula IV, which is an adamantane-based scaffold. 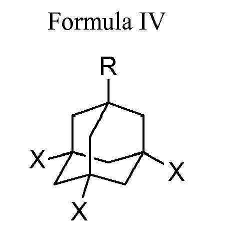 with X and R as described for Formula II.

Other molecular constructs like dendrimeric structures containing the above described substituents and tags are possible.

The group X as described above that is covalently bound to a cyclic polyamine unit, such as cyclen or cyclam can be bound either to a C or, preferably, to one of its N atoms.

In another preferred embodiment, the bridging unit can be a unit according to formula V or Va, which are peptide-based scaffolds. Formula V is based on a peptidic scaffold with α-amino acids, whereas formula Va is based on a peptidic scaffold with β-amino acids. 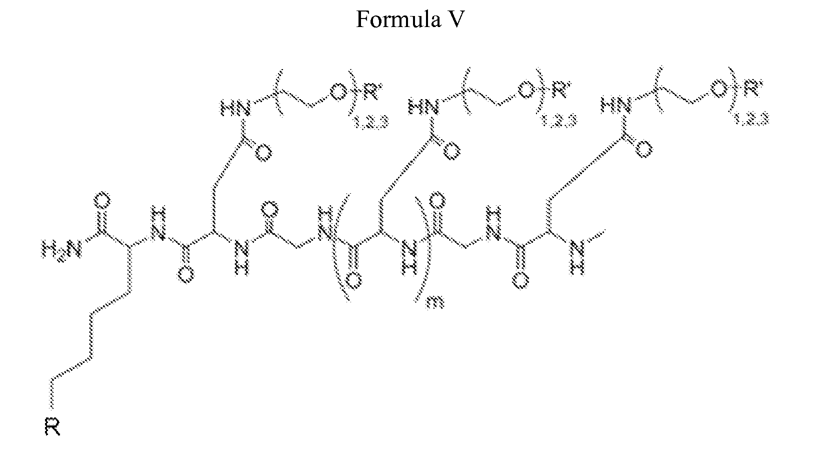 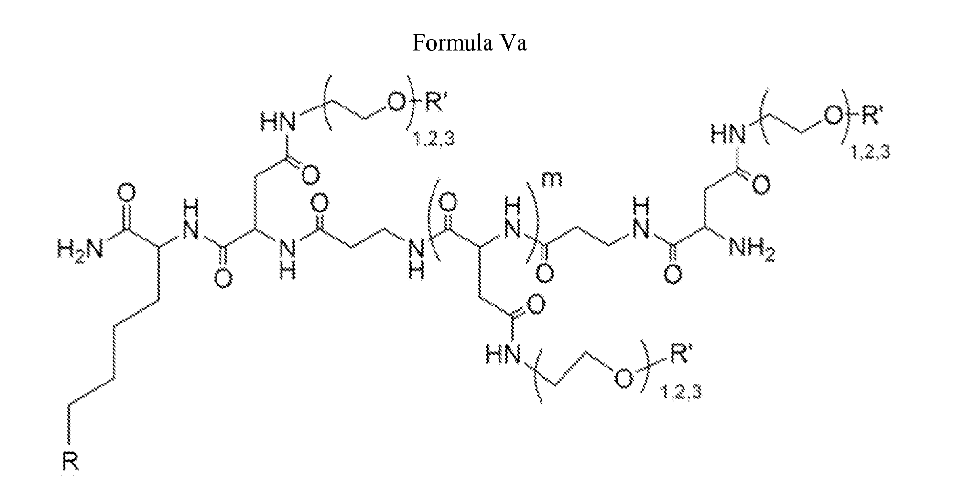 wherein m = 1, 2, 3, 4, 5, or 6, and
R' = an alkyl or aryl bridge connected with a cyclic polyamine unit of formula Ia or formula Ib, and
R = a detectable tag as described above.

The cyclic polyamine unit, such as cyclen or cyclam, can be bound either via a C or, preferably, via one of its N atoms in a compound of formula V or Va.

Furthermore, it is possible to provide functionalized particles like ultrasmall superparamagnetic iron oxide particles (USPIOs) to which the cyclen or cyclam or mixures thereof of the compounds of the invention are bound to predefined surfaces. Methods of binding such functionalized particles to the compound have been described elsewhere (27).

By using the Zn²⁺ complexed and FITC-labeled bis-cyclen derivative shown in figure 3 in binding experiments with control and apoptotic Jurkat cells, the inventors surprisingly obtained results comparable to thoses obtained with fluorescent annexin V. The FACS measurements were reproducible opposing the experiments performed with Zn²⁺ complexed and FITC-labeled bis-DPA complexes.

The positive results acquired with the in vitro apo-test system (15) show that this class of small metal-organic compounds is usefull for apoptosis imaging. Table 1 lists preferred compounds of the invention. Moreover, prefered specific compounds of formulas VI, VII, and VIII are given below.

In another aspect of the invention, a compound as described herein is used for binding and detecting phosphatidylserine, both in vitro and in vivo. In particular, such use makes apoptosis imaging in vivo possible, such as using positron emission tomography (PET), single-photon emission computed tomography (SPECT), magnetic resonance imaging (MRI), and/or optical imaging.

Advantageously, such a method allows for the assessment of a response of a patient suffering from a tumor, from an infection, and/or from an inflammation, to an anti-tumor therapy, to anti-infection therapy and/or to anti-inflammatory therapy, respectively. In particular, the compound of the invention is advantageous as the decrease in its uptake is readily detectable.

Accordingly, a method for detecting phosphatidylserine and thereby the progression or regression of a tumor, of an infection, and/or of an inflammation in a tissue of a patient is also provided. Such a method comprises or contains the following steps:
a) administering a compound as described herein to a patient, and
b) measuring the amount and/or the concentration of the compound in the tissue.
The patient can be any mammal and is preferably a human being. The measurement of the amount and/or the concentration of the compound in the tissue of interest, i.e. the diseased tissue, can be performed using PET, SPECT, MRI, and/or optical imaging.

A compound used in this detection method preferably comprises at least two cyclic polyamine units that are connected via a bridging unit for connecting at least two cyclic polyamine units with each other. The bridging unit is preferably selected from the group consisting of the compounds of formulas II to V as shown above.

Furthermore, a method for synthesizing of a compound as described herein is also provided. According to this method, at least two cyclic polyamine units are connected via a bridging unit for connecting at least two cyclic polyamine units with each other. Preferably, the bridging unit is selected from the group consisting of the compounds of formulas II to V.

In a further aspect, the invention relates to a pharmaceutical composition comprising a compound as described herein, and possibly further a suitable carrier and/or additive.

In a further aspect, the invention relates to the use of cyclic polyamine compound as described herein or composition thereof for in-vivo or in-vitro detection of apoptosis. More preferably, the use is for in-vitro detection. In-vitro detection can be conducted by FACS, microspectrometry or ELISA.

In yet a further aspect, the invention relates to a kit for binding and detecting phosphatidylserine, particularly in vitro. Such a kit comprises a compound as described herein and appropriate buffers for dissolving and/or diluting the compound, an agent such as an antibody for detecting the compound and/or ELISA plates for performing an ELISA assay.

### Figures

**Figure 1****:** Illustration of the interaction in one of domain III of annexin V with glycerophosphoserine (truncated PS) on Ca²⁺-annexin V binding sites (9). Primary AB and secondary AB' Ca²⁺ sites are located within the loop between the A and B helices of each domain. They are also called type II and type III Ca²⁺ binding sites, respectively. The structural alignment of glycerophosphoserine (light) in AB and AB' Ca2⁺-binding sites in third domain of annexin V (shaded) is shown (28).
**Figure 2**
   The depiction of a FITC labeled Zn²⁺bis-DPA complex according to the state of the art.
**Figure 3**
   The depiction of a prefered compound of the invention, namely a FITC labeled Zn²⁺bis-cyclen complex.

**Table 1.** Preferred compounds of the invention: Each compound shown comprises at least two cyclic polyamine units in the form of a cyclen or cyclam, joined by a bridging unit. The bridging unit may also carry a detectable tag R, which may be a labeled substituent R₂ that can, optionally, comprise a spacer R₁. Any given bridging unit given below can be combined with any combination of R₁ and R₂ written in the same portion of the table (separated by bold lines).
The preferred compounds are shown here complexed with Zn²⁺. It is possible, however, that the compounds form a complex with any other multivalent (in particular a bi- or trivalent) metal ion as described herein, or with mixtures thereof.

| **Cyclic polyamine units joined by a bridging unit** | **R** | |
|---|---|---|
| | **R₁** | **R₂** |
| 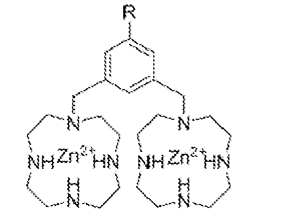 | 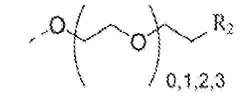 | 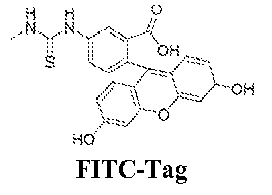 |
| | 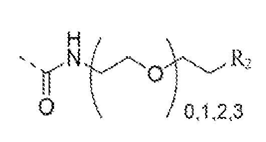 | 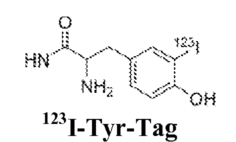 |
| | | 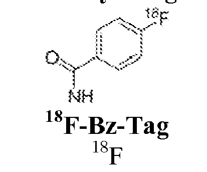 |
| 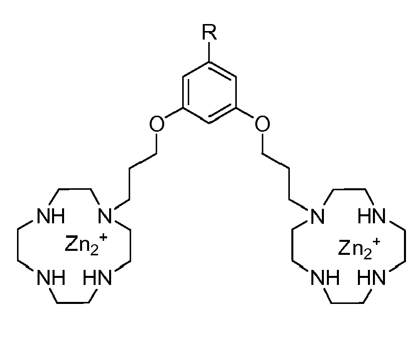 | 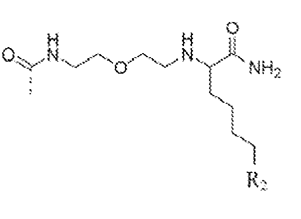 | ¹⁸F FITC-Tag ¹²³I-Tyr-Tag ¹⁸F-Bz-Tag |
| | 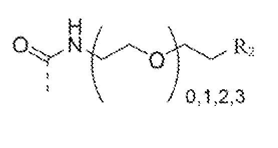 | |
| 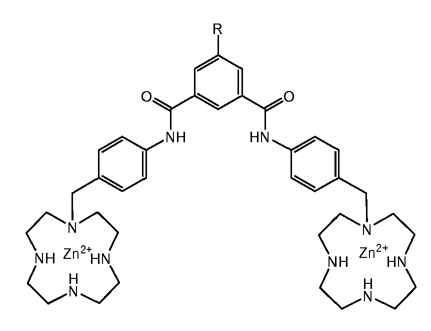 | 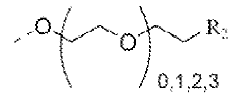 | ¹⁸F FITC-Tag ¹²³I-Tyr-Tag ¹⁸F-Bz-Tag |
| | 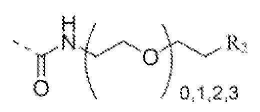 | |
| 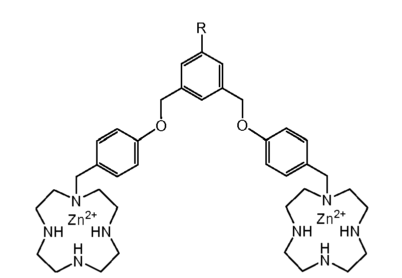 | 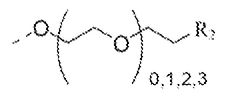 | ¹⁸F FITC-Tag ¹²³I-Tyr-Tag ¹⁸F-Bz-Tag |
| 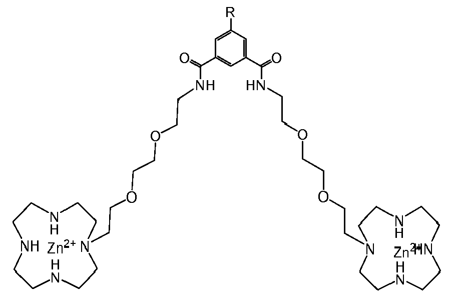 | 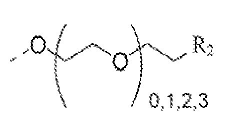 | ¹⁸F FITC-Tag ¹²³I-Tyr-Tag ¹⁸F-Bz-Tag |
| 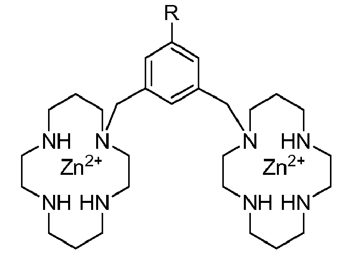 | 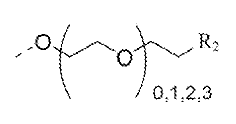 | ¹⁸F FITC-Tag ¹²³I-Tyr-Tag ¹⁸F-Bz-Tag |
| 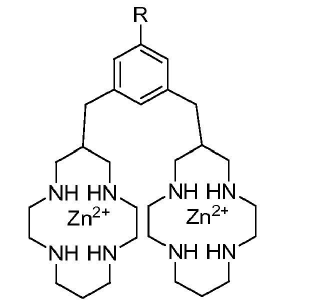 | 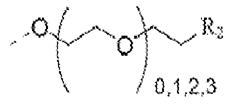 | ¹⁸F FITC-Tag ¹²³I-Tyr-Tag ¹⁸F-Bz-Tag |
| 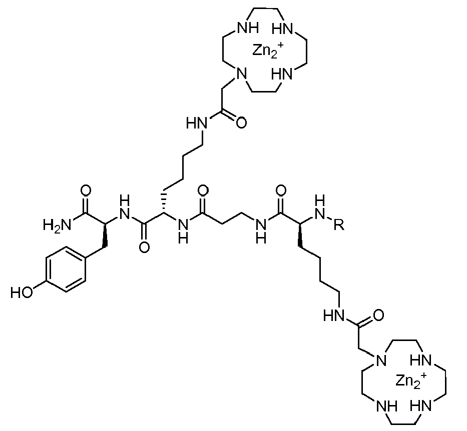 | 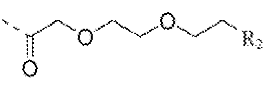 | ¹⁸F FITC-Tag ¹²³I-Tyr-Tag ¹⁸F-Bz-Tag |
| 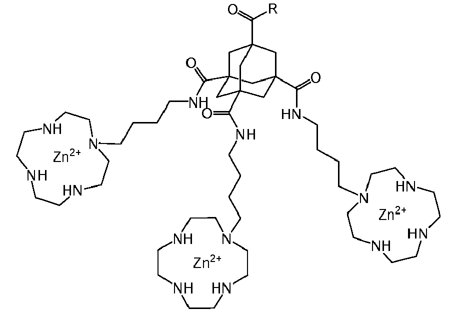 | 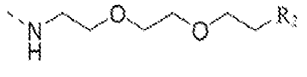 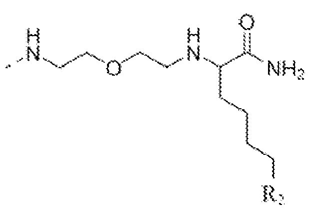 | ¹⁸F FITC-Tag ¹²³I-Tyr-Tag ¹⁸F-Bz-Tag |
| 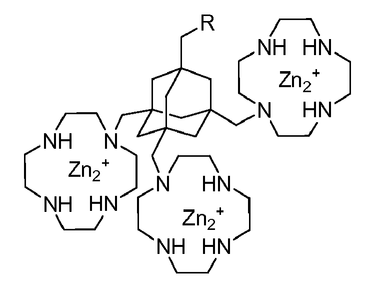 | 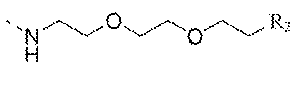 | ¹⁸F FITC-Tag ¹²³I-Tyr-Tag ¹⁸F-Bz-Tag |
| 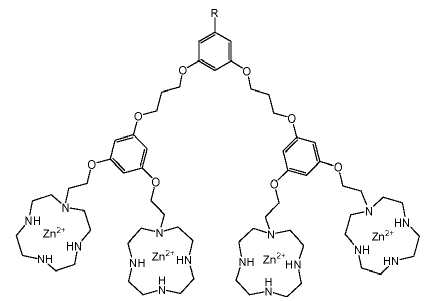 | 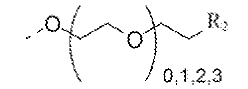 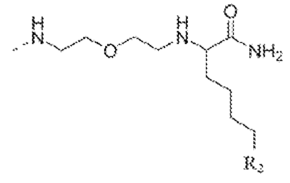 | ¹⁸F FITC-Tag ¹²³I-Tyr-Tag ¹⁸F-Bz-Tag |
| 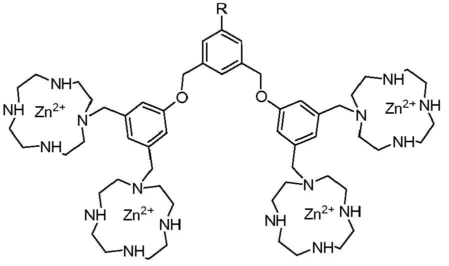 | 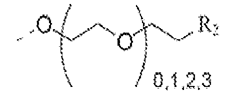 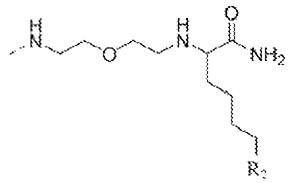 | ¹⁸F FITC-Tag ¹²³I-Tyr-Tag ¹⁸F-Bz-Tag |
| 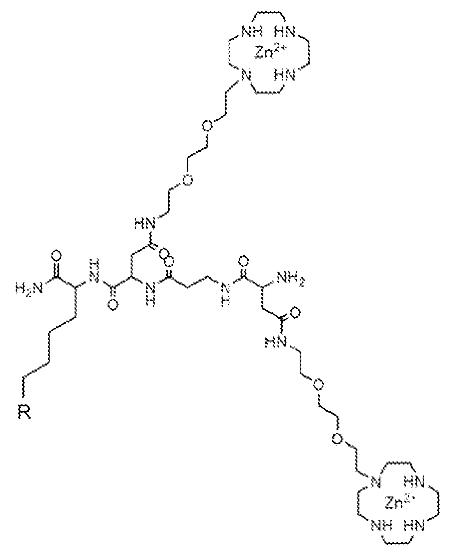 | | ¹⁸F FITC-Tag ¹²³I-Tyr-Tag ¹⁸F-Bz-Tag |
| 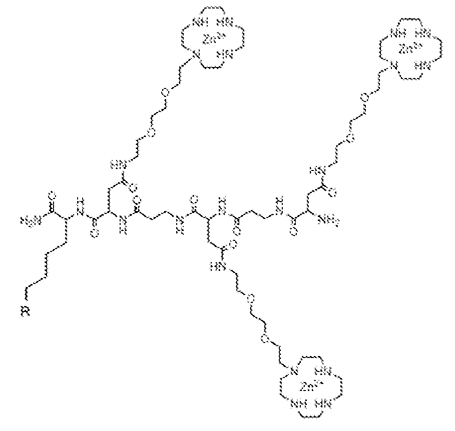 | | ¹⁸F FITC-Tag ¹²³I-Tyr-Tag ¹⁸F-Bz-Tag |
| 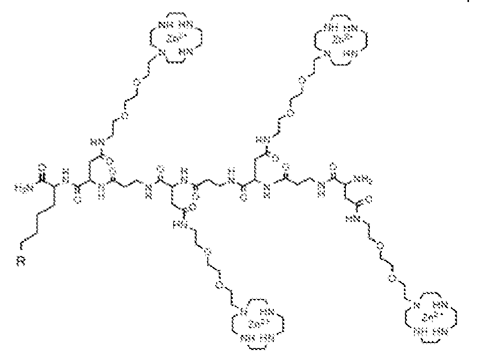 | | ¹⁸F Tag FITC-Tag ¹²³I-Tyr-Tag ¹⁸F-Bz-Tag |

**Table 2.** Preferred compounds of the invention: Each compound shown comprises at least two cyclic polyamine units in the form of a cyclen or cyclam, joined by a bridging unit. The bridging unit may also carry a residue A. Z is a radiohalogen for detecting the compound. The cyclic polyamine unit joined by the bridging unit given below can be combined with any combination of residue A and Z in the table.
The preferred compounds are shown here complexed with Zn²⁺. It is possible, however, that the compounds form a complex with any other multivalent (in particular a bi- or trivalent) metal ion as described herein, or mixtures thereof.

| **Cyclic polyamine units joined by a bridging unit** | **A** | **Z** |
|---|---|---|
| 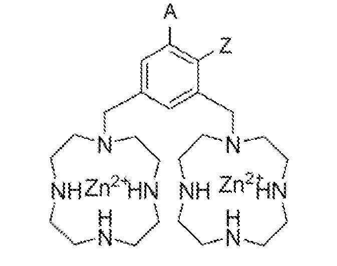 | OH, alkyloxy, aryloxy | ¹²³I, ¹²⁴I, ¹⁸F |

Preferred, specific compounds of the invention:

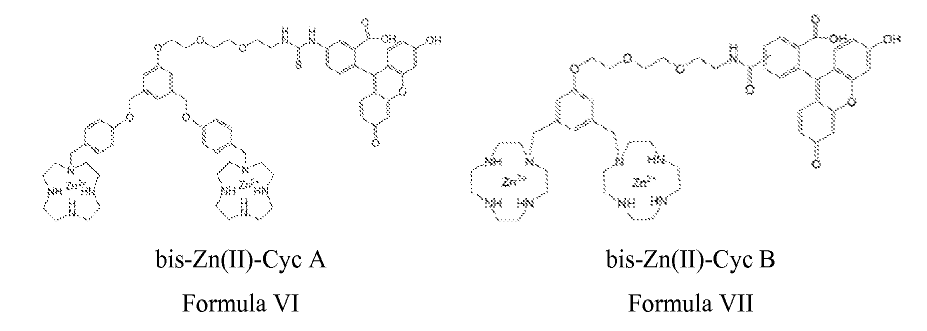

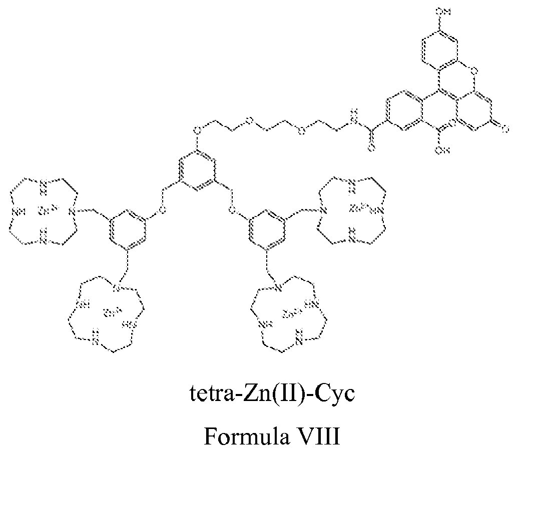

### Examples

### a) Synthesis of fluorescence-labeled Bis-Cyclen complexes

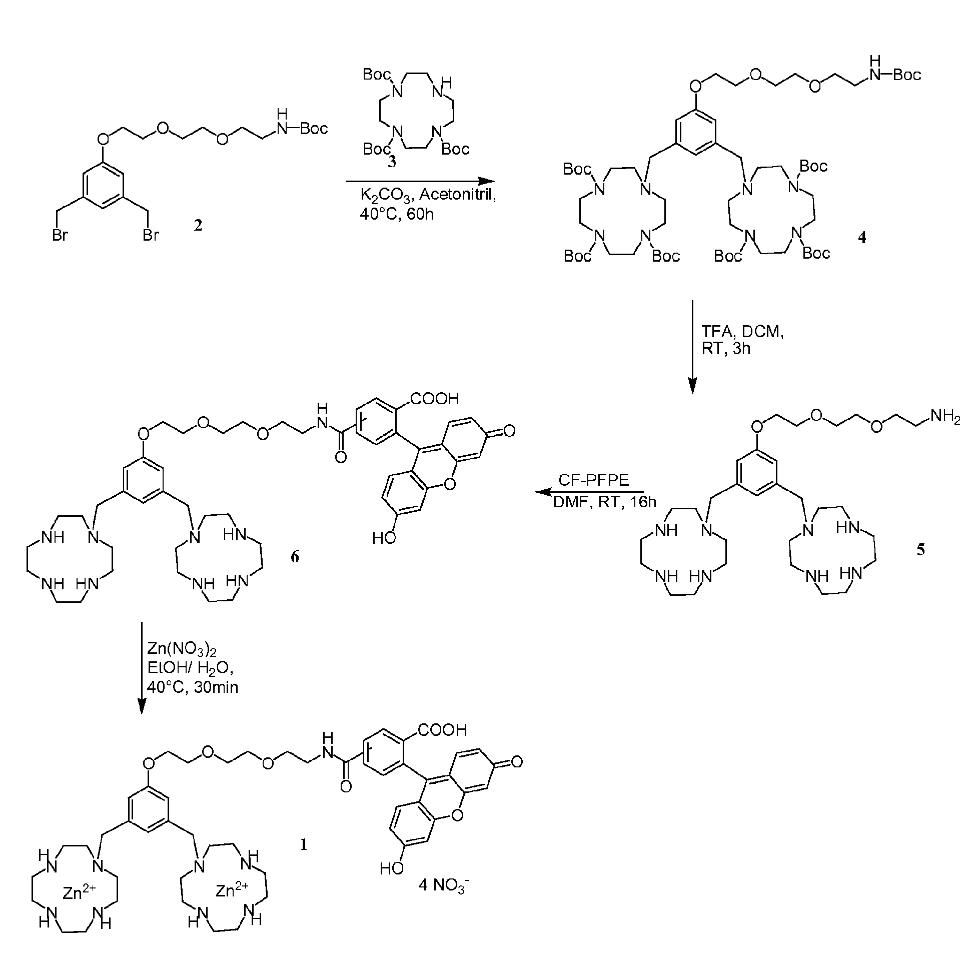

All solvents used were bought as water-free p. A.-solvents. Silica gel 60 (0.04 - 0.063 µm, Fluka) was used for the column chromatography purification. The preparative HPLC- purification was performed with an HPLC by Agilent (1100 Series) using the semi-preparative column Chromolith® SemiPrep, RP18e, 100-10 mm (Merck).

### Synthesis of Compound 4

The bromo compound **2** (22) (130 mg, 254 µmol) and 1,4,7-tris(*tert*-butyloxycarbonyl)cyclen **(3)** (23) (295 mg, 624 µmol, 2.5 eq.) were dissolved in 4 ml acetonitril and potassium carbonate (175 mg, 1.27 mmol) was added. The suspension was stirred for 60 h at 40 °C. Subsequently, the potassium carbonate was removed by filtering and the solvent was removed *i. vac.* Following column chromatography purification using silica gel with EtOAc/petrol ether = 1:1 as eluent, a white solid substance was obtained.
Yield: 220 mg (90 %)

### Synthesis of Compound 5

The biscyclen compound **4** (220 mg, 170 µmol) was dissolved in 1.4 ml 50 % TFA in dichloromethane and stirred for 3 h at room temperature. The solvent was removed *i. vac.* No further purification was performed.
Yield: quantitatively

### Synthesis of Compound 6

The amine **5** (8.3 mg, 13.9 µmol) was dissolved in 250 µl DMF anhydr. 5-/6-carboxyfluoresceinpentafluorophenylester (24) (7.5 mg, 13.9 µmol) was also dissolved in DMF anhydr. and added to **5.** The reaction mixture was stirred for 16 h at room temperature. The solvent was removed *i. vac.* The raw product was purified at the semi-preparative HPLC (A = H₂O, B = ACN, 0.1 % TFA; 5 min 40 % B).
MALDI-MS: m/z = 968.6 (M+H⁺), 990.6 (M+Na⁺) (calc 967.55)

### Synthesis of Compound 1

The ligand **6** (1.1 mg, 1.16 µmol) was dissolved in 20 µl EtOH and 23.1 µl of an aqueous 0.1 mol/l Zn(NO₃)₂-solution (2.31 µmol). The solution was heated for 40 minutes to 50 °C. After lyophilizing a yellow solid substance was obtained.
Yield: 100%

### Flow cytometry (FACS)

FACS measurements were performed with a DAKO Galaxy Flow Cytometry System using the following experimental setup: Jurkat-cells were grown to a density of about 1*10⁶ cells/ml. Apoptosis was induced by adding 1 µmol/l staurosporine. After incubation for 4 h at 37 °C, the cells were washed with cold PBS-buffer (140 mmol/l NaCl, 10 mmol/l KCl, 6.4 mmol/l Na₂HPO₄, 2 mmol/l KH₂PO₄) and resuspended in cold TES-buffer (5 mmol/l TES, 145 mmol/l NaCl). 10⁶ cells in 100 µl buffer with 1 µl of a 0.5 mmol/l aqueous solution of complex **1** were incubated for 15 minutes at 37 °C. Subsequently, cells were washed twice with 500 µl TES-buffer and the cells were resuspended in 400 µl TES-buffer. 100 µl of the probe were diluted with 900 µl of buffer and measured immediately. To discriminate against necroses, 10 µl propidiumiodide solution (250 µg/ml) were added directly before the measurement.

**Table 2. Examples for apoptosis detection by Annexin V and three different Zn(II)-complexes on staurosporine treated Jurkat cells, using propidium iodide (PI) for necrosis discrimination.**

| Apoptosis Sensor | % apoptosis (green) | % necrosis (PI) (red) | % necrosis/ late apoptosis (red+green) |
|---|---|---|---|
| Anx V | 22 | 18 | 25 |
| bis-Zn(II)-Cyc A | 32 | 25 | 15 |
| bis-Zn(II)-Cyc B | 34 | 24 | 18 |
| tetra-Zn(II)-Cyc | 30 | 25 | 13 |

Examples for apoptosis detection with Annexin V and three different Zn(II)-complexes on staurosporine treated Jurkat cells, using propidium iodide (PI) for necrosis discrimination are summarized in Table 2. The results demonstrate the parallelism of apoptosis-detection potency of the three Zn(II)-Cyclene derivatives with Annexin V.

### b) Synthesis of radioactively-labeled bis-cycle complexes

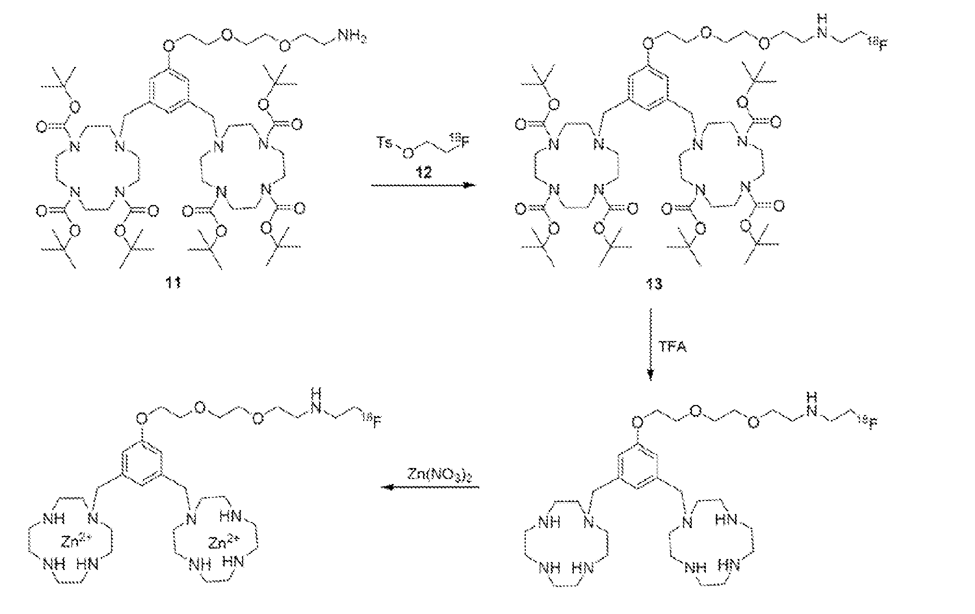

### Synthesis of Compound 11

Compound **5** (10 mg, 16.8 µmol) was dissolved in 1 ml EtOH and 353 µl of an aqueous 0.1 mol/l ZnNO₃-solution (353µmol) was added. The solution was heated for 60 minutes at 50 °C. After lyophilization, a colorless solid substance was obtained.
Yield: 100%.

### Synthesis of Compound 13

1 mg of the Zn²⁺-complex **11** was dissolved in 0.5 ml CH₃CN and reacted with the previously synthesized 2-[¹⁸F]fluoroethyltosylat **12** at 40 °C at 10 minutes. Synthesis of the precursor **12** was performed according to the literature (25). The reaction mixture was run over an RP18 HPLC.
Radiochemical yield: 12 %

The product **13** was then used in an apoptosis assay as described (22). The compound was also analyzed in an animal experiment regarding its pharmaceutical kinetics. A test regarding the incorporation into a tumor with a therapeutically induced apoptosis was also performed.

### Apoptosis-Assay

*Cell Uptake Measurements.* The cell uptake measurements were performed with staurosporine treated Jurkat cells and untreated controls. Before commencing the cell uptake measurements control cells and staurosporine treated cells were examined by flow cytometry for apoptotic cell fractions using annexin V-Alexa Fluor 488 (Molecular Probes). The cell counts were 5×10⁶ cells/350 µl RPMI 1640. Treated and control cells were incubated at 37 °C with 0.2 MBq of the respective radiolabeled Zn²⁺ complex **13.** The specific activities of the compounds were no-carrier added. After 10, 15, 20, 30, and 60 min the tubes were briefly vortexed and 10 µl samples of the control and apoptotic cells were layered on top of a 400 µl Eppendorf microcentrifuge tube containing 350 µl of a 75:25 mixture of silicon oil, density = 1.05 (Aldrich), and mineral oil, density 0.872 (Acros) (21). Instantaneous centrifugation at 15,000 rpm for 2 min afforded the complete separation of cellular radioactivity from the medium. After freezing the tubes with liquid nitrogen the bottom tips containing the cell pellet were cut off. The cell pellets and the supernatants were counted in a γ-counter. The cell uptake was calculated as % uptake = 100 × cpm (pellet) / [cpm (pellet) + cpm (supernatant)]. There was binding of complex **13** to apoptotic cells, while no binding was observed in untreated control cells.

### Pharmacokinetics

### Animals and tumor growth

5 × 10⁶ cells of 38C13 murine B cell lymphomas were suspended in PBS subcutaneously inoculated into anterior region of the mouse trunk of female 7-8-week-old BALB/c nu/nu mice (Charles River WIGA, Sulzfeld, Germany) and the tumors were allowed to grow for about 10 to 14 days until approximately 1 cm³ in size. The animals underwent treatment with 100 mg/kg of cyclophosphamide injected i.p. For other models male nu/nu mice (0.5 - 1 MBq per mouse), heterotransplanted with either FRO82-2 thyroid or MCF-7 breast tumors were used.

### Organ distribution with ¹⁸F-labeled Zn²⁺ complex 13

Mice were injected i.v. with 25-50 mg/kg of ^{99m}Tc HYNIC annexin V or complex **13** (100-150 mCi/animal) 20 hr after cyclophosphamide administration. Animals were then either imaged and sacrificed 1 hr after injection of radiopharmaceutical after tumor removal for scintillation counting and histopathologic studies. Or the radiolabeled compound was injected via the tail vein and at 5 min, 15 min, 45 min and 135 min p.i. the animals were sacrificed. Blood, heart, lung, spleen, liver, kidney, muscle, brain and the tumor were dissected, blotted dry and weighed.
The radioactivity was measured with a γ-counter (CobraII, Canberra Packard, Meriden, USA) along with a sample of the injection solution to calculate the percentage of injected dose per gram of tissue (%ID/g) of the tissues.
Annexin V and compound **13** showed accumulation in apoptotic areas of the tumors.

### Abbreviations

ACN - Acetonitril
CF-PFPE - 5-/6-Carboxyfluoresceinpentafluorphenylester
DCM - Dichloromethane
DMF - N,N-Dimethylformamide
EtOAc - Ethyl acetate
EtOH - Ethanol
FACS - Fluorescence-activated cell sorting
FITC - Fluorescein
TES - [*N*-[Tris(hydroxymethyl)methyl]-2-aminosulfonic acid]
PI - Propidium iodide
TFA - Trifluoro acetic acid

### References

1. Blankenberg FG, Katsikis PD, Tait JF et al. Increased localisation of Tc-99m hydrazino nicotinamide (HYNIC) labeled annexin V lipocortin in lymphatic tissue of animals treated with dexamethasone [abstract]. J Nucl Med. 1997; 38(suppl):268P.
2. Blankenberg FG, Katsikis PD, Tait JF et al. In vivo detection and imaging of phosphatidylserine expression during programmed cell death. Pro Natl Acad Sci U S A. 1998;95:6349-6354.
3. Koopman G, Reutelingsperger CP, Kuijten GA, Keehnen RM, Pals ST, van Oers MH. Annexin V for flow cytometric detection of phosphatidylserine expression on B cells undergoing apoptosis. Blood. 1994;84:1415-1420.
4. Bohn H, Kraus W. Isolation and characterization of a new placenta specific protein (PP10). Arch Gynecol. 1979;227:125-134.
5. Reutelingsperger CP, Hornstra G, Hemker HC. Isolation and partial purification of a novel anticoagulant from arteries of human umbilical cord. Eur J Biochem. 1985;151:625-629.
6. Rescher U, Zobiack N, Gerke V. Intact Ca(2+)-binding sites are required for targeting of annexin 1 to endosomal membranes in living HeLa cells. J Cell Sci. 2000;113:3931-3938.
7. Boon JM, Smith BD. Facilitated phosphatidylcholine flip-flop across erythrocyte membranes using low molecular weight synthetic translocases. J Am Chem Soc. 2001;123:6221-6226.
8. Andree HA, Stuart MC, Hermens WT, Reutelingsperger CP, Hemker HC, Frederik PM, Willems GM. J Biol Chem 1992 Sep 5;267(25):17907-12.
9. Tait JF, Gibson D, Fujikawa K. Phospholipid binding properties of human placental anticoagulant protein-1, a member of the lipocortin family. J Biol Chem. 1989;264:7944-7949.
10. Swairjo MA, Concha NO, Kaetzel MA, Dedman JR, Seaton BA. Ca(2+)-bridging mechanism and phospholipid head group recognition in the membrane-binding protein annexin V. Nat Struct Biol. 1995;2:968-974.
11. Tait JF, Gibson DF, Smith C.Measurement of the affinity and cooperativity of annexin V-membrane binding under conditions of low membrane occupancy. Anal Biochem. 2004; 329: 112-119.
12. Nicolson DW, Thornberry NA. Caspases: killer proteases. TIBS. 1997; 22: 299-306.
13. Cohen GM. Caspases: the executioners of apoptosis. J Biochem. 1997; 326: 1-16.
14. Thornberry NA, Lazebnik Y. Caspases: enemies within. Science. 1998; 282: 1312-1316.
15. Haberkorn U, Kinscherf R, Krammer PH, et al. Investigation of a potential scintigraphic marker of apoptosis: radioiodinated Z-Val-Ala-DL-Asp(o-methyl)-fluoromethylketone. Nucl Med Biol. 2001; 28: 793-798.
16. Bauer C, Bauder-Wuest U, Mier W, Haberkorn U, Eisenhut M. Investigation of 131I labelled Peptides as Caspases Substrates for Apoptosis Imaging. J Nucl Med 2005; 46: 1066-1074.
17. Kamp D, T. Sieberg and C.W. M. Haest, Biochemistry, 2001, 40, 9438.
18. Zweifach A, Biochem. J., 2000, 349, 255.
19. Dachary-Prigent J, J. M. Pasquet, J. M. Freyssinet and A. T. Nurden, Biochemists, 1995,34,11625.
20. Hanshaw RG, E. J. O'Neil, M. Foley, R. T. Carpenter and B. D. Smith, J. Mater. Chem., 2005, 15, 2707.
21. Hanshaw RG, C. Lakshmi, T. N. Lambert, J. R. Johnson and B. D. Smith, ChemBio-Chem, 2005, 12, 2214.
22. Lakshmi C, R. G. Hanshaw, B. D. Smith, Tetrahedron 2004, 60, 11307-11315.
23. Peng W et al., Bioorg. Chem. 2005, 33, 374-385.
24. Adamczyk M, Fishpaugh JR, Heuser KJ. Bioconjugate Chem. 1997, 8, 253-255.
25. Wester HJ, Herz M, Weber W, Heiss P, Senekowitsch-Schmidtke R, Schwaiger M, Stocklin G. Synthesis and radiopharmacology of O-(2-[18F]fluoroethyl)-L-tyrosine for tumor imaging. J Nucl Med 1999 Jan;40(1):205-12.
26. Bauer C, Bauder-Wuest U, Mier W, Haberkorn U, Eisenhut M. Investigation of 131I labelled Peptides as Caspases Substrates for Apoptosis Imaging. J Nucl Med 2005; 46: 1066-1074.
27. Chunfu Z, Jugold M, Woenne EC, Lammers T, Morgenstern B, Mueller MM, Zentgraf H, Bock M, Eisenhut M, Semmler W, and Kiessling f. Specific Targeting of Tumor Angiogenesis by RGD-Conjugated Ultrasmall Superparamagnetic Iron Oxide Particles Using a Clinical 1.5-T Magnetic Resonance Scanner. Cancer Res 2007; 67: 1555-1562.
28. Eisenhut M., U. Haberkorn. Invited perspective: The molecular position of radiolabels and its impact on functional integrity of proteins. J Nucl Med 2006; 47: 1400-1402

## Claims

1. A compound for binding phosphatidylserine,
**characterized in that**
the compound comprises at least one cyclic polyamine unit for the complexation of a bi- or trivalent metal ion.

2. The compound according to claim 1, wherein the bivalent metal ion is chosen from the group consisting of Zn²⁺, Ca²⁺, Cu²⁺, Be²⁺, Mg²⁺, Sr²⁺, Ba²⁺, Cd²⁺, Cr²⁺, Mn²⁺, Fe²⁺, Co²⁺, Ni²⁺, Pb²⁺, Hg²⁺, and corresponding radiometals of the aforementioned ions.

3. The compound according to claim 1, wherein the trivalent metal ion is chosen from the group consisting of Al³⁺ G³⁺, In³⁺, C³⁺, Mn³⁺, Fe³⁺, Co³⁺, Ni³⁺, and correspond-ing radiometals of the aforementioned ions.

4. The compound according to claims 1 to 3, further comprising a tag for detecting the compound.

5. The compound according to claims 1 to 4, wherein the tag is chosen from the group consisting of fluorescent dyes, enzymes, proteins, and radioactive labels.

6. The compound according to claim 5, wherein
- the fluorescent dye is chosen from the group consisting of fluoresceins, rhodamines, BODIPY, Cy-3, Cy-5, and derivatives thereof.
- the protein is chosen from the group consisting of peroxidases, luziferase, biotin, avidin, and antibodies or parts thereof;
- the radioactive label is chosen from the group consisting of ^{99m}Tc, ¹⁸F, ¹¹C, ¹²⁴I, ¹²³I, ⁶⁴Cu²⁺, ⁶⁸Ga³⁺, ¹¹¹In³⁺, ¹⁴C, ³H, ³²P and ³³P_{.}

7. The compound according to claims 1 to 6, wherein the cyclic polyamine unit is cyclen with a structure of formula Ia or cyclam with a structure of formula Ib.

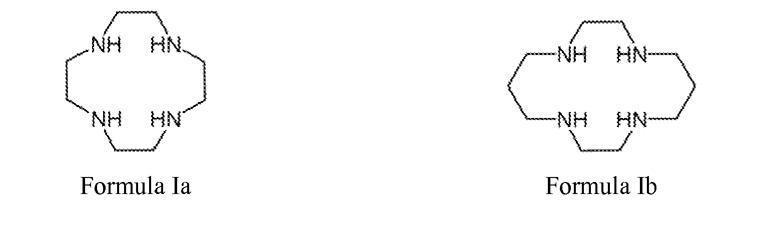

8. The compound according to claims 1 to 7, comprising two cyclic polyamine units that are connected via a bridging unit, that is selected from a group consisting of
(a) 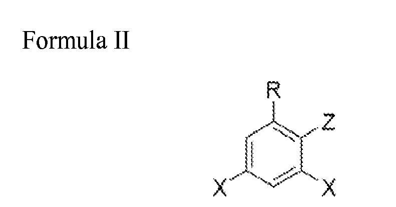 with
X = (CH₂)ₙ, (CH₂CH₂O)ₙ, or CH₂OPhCH₂, or CO,
wherein n=1, 2, or 3, and Ph = phenyl,
wherein X is each covalently bound to a cyclic polyamine unit; and
Z = a halogen, a radiohalogen (such as ¹⁸F, ¹²³I), or H; and
R = H, or a tag as described in claim 4-6, preferably a fluorescent dye, ¹⁸F, ¹¹C, ¹²³I, or a labeled substituent,
wherein the tag is preferably a labeled substituent R₂,
with R₂ = 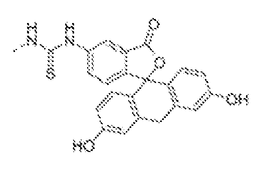 or 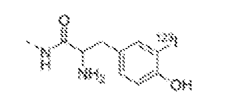 or 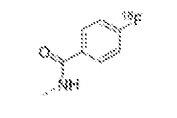 or 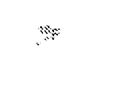 which optionally comprises a spacer R₁, such that the labeled substituent R₂ with the spacer R₁ is 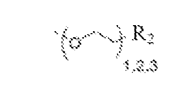 or 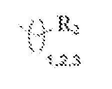
(b) a substituted benzene-based scaffold of formula III 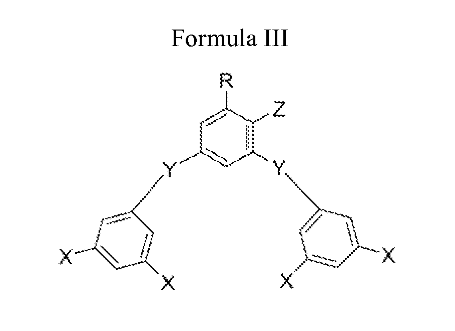 with
Y = (CH₂)ₙ with n=1, 2, or 3, (CH₂CH₂O)ₘ with m=0, 1, 2, or 3 O(CH₂)ₚO (with p=1, 2, 3, or 4), or O (independently of each other), or mixtures thereof; and
Z = a halogen, a radiohalogen (such as ¹⁸F, ¹²³I), or H; and
R and X as described for formula II above;
(c) an adamantane-based scaffold of formula IV 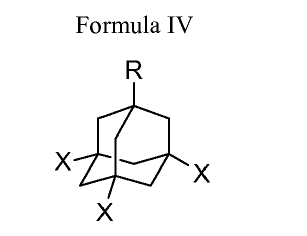 with X and R as described above for formula II;
(d) a peptide-based scaffold of formula V (peptidic scaffolds with α-amino acids) or formula Va (peptidic scaffolds with β-amino acids) 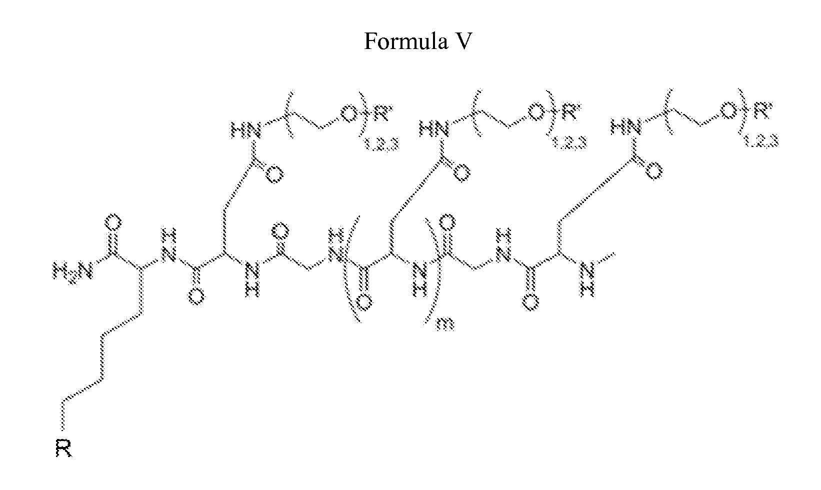 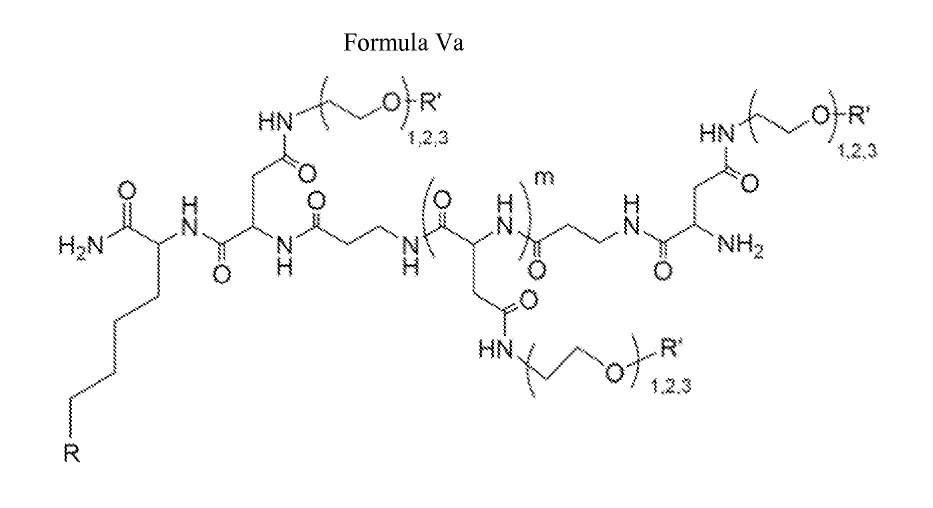
wherein m = 1, 2, 3, 4, 5, or 6, and
with R' = an alkyl or aryl bridge connected with a cyclen or a cyclam, independently of each other; and
with R as described for formula II.

9. The compound according to claims 1 to 8, as shown in table 1 or table 2, or of formula VI, VII, or VIII.

10. Use of a compound according to claims 1 to 9 for binding phosphatidylserine.

11. Use of a compound according to claims 1 to 9 for apoptosis imaging.

12. Use according to claim 10 or 11, using positron emission tomography (PET), single-photon emission computed tomography (SPECT), magnetic resonance imaging (MRI), or optical imaging.

13. Use according to claims 10 to 12, for assessing the status of a tumor or the response of a patient suffering from a tumor, from an infection, and/or from an inflammation, to an anti-tumor therapy, to anti-infection therapy and/or to anti-inflammatory therapy, respectively.

14. A method for detecting the progression of a tumor, of an infection, and/or of an inflammation in a tissue of a patient, comprising
a) administering a compound according to claims 1 to 9 to a patient, and
b) measuring the amount or the concentration of the compound in the tissue.

15. A method for synthesizing of a compound according to claims 1 to 9, wherein at least two cyclic polyamine units are covalently bound to each other via a bridging unit.

16. The method according to claim 15, wherein the bridging unit is selected from the formulas II, III, IV, V, Va.

17. A pharmaceutical composition, comprising a compound of claims 1 to 9.

18. The pharmaceutical composition according to claim 17, further comprising a suitable carrier and/or additive.

19. A kit for binding phosphatidylserine, comprising a compound of claims 1 to 9, and optionally buffers for detecting phosphatidylserine in vitro.

20. Use of a compound according to claims 1 to 9 or pharmaceutical composition according to claim 17 for in-vivo or in-vitro detection of apoptosis.
